# EUROPEAN PATENT SPECIFICATION

(11) **EP 3 861 971 B1**
(45) Date of publication and mention of the grant of the patent: **27.12.2023**
(21) Application number: 19869434.1
(22) Date of filing: 10.09.2019
(51) Int. Cl.: A61F 13/511, A61F 13/15, A61F 13/47, A61F 13/515, A61F 13/535, A61F 13/539

(54) **ABSORBENT ARTICLE**
ABSORBIERENDER ARTIKEL
ARTICLE ABSORBANT

(30) Priority: 01.10.2018 JP 2018186967
(43) Date of publication of application: 11.08.2021
(73) Proprietor: Kao Corporation, Chuo-Ku Tokyo 103-8210 (JP)
(72) Inventor: KAWAGUCHI, Hiroko, Haga-gun, Tochigi 321-3497 (JP); TADAI, Tomohiro, Haga-gun, Tochigi 321-3497 (JP)
(74) Representative: Vossius & Partner Patentanwälte Rechtsanwälte mbB
(86) International application number: PCT/JP2019/035527
(87) International publication number: WO 2020/071064

(56) References cited:
- WO-A1-2013/129332
- WO-A1-2016/098867
- WO-A1-2017/187778
- WO-A1-2017/187778
- JP-A- 2002 045 391
- JP-A- 2007 195 665
- JP-A- 2015 101 817
- JP-A- 2015 104 650
- JP-A- 2015 104 650
- JP-A- 2015 518 091
- JP-A- 2016 501 669
- US-B2- 6 800 789

## Description

### Technical Field

The present invention relates to absorbent articles, such as disposable diapers and sanitary napkins.

### Background Art

There have been proposed absorbent articles, such as disposable diapers, that include a skincare agent provided to a member constituting the absorbent article from the viewpoint of suppressing skin trouble, such as skin rashes. For example, Applicant has previously proposed an absorbent article wherein a plant extract having skincare effects and having substantially no decomposition effect on superabsorbent polymers is applied to a predetermined section of the absorbent article, the plant extract being present in a state where it can be transferred from the predetermined section onto the wearer's skin and adhere thereto (JP 2002-045391A). The plant extract is applied to a top layer that sandwiches and holds an absorbent layer together with a leak-proof layer.

JP 2018-15520A discloses an absorbent article including a topsheet that has a base portion and projections protruding from the base portion toward the skin-facing surface side, wherein the apex and side walls of each projection contain a weak-acid agent including plant-derived components, and the fiber density of the side walls is lower than the fiber density of the apex. According to JP2018-15520A, the weak-acid agent is applied to the surface on the skin-facing surface side of the topsheet. Further absorbent articles are disclosed in JP2015-104650A, WO2017/187778A1 and US6800789B2.

### Summary of Invention

The invention relates to an absorbent article as defined in independent claim 1.

According to a preferred embodiment the skincare agent is a plant extract. More preferably, the skincare agent is a hamamelis extract.

According to a preferred embodiment wherein the skincare agent is provided to a constituent fiber of the topsheet.

According to a preferred embodiment the absorbent article includes a pair of leak-proof cuffs provided respectively to both lateral sides along a longitudinal direction on a skin-facing surface of the topsheet; and the weakly-joined central region is located between the pair of leak-proof cuffs

According to a preferred embodiment when worn, the weakly-joined central region is located in opposition to both a wearer's urinary excretion section and fecal excretion section.

According to a preferred embodiment the topsheet is provided with the skincare agent in a region overlapping the weakly-joined central region.

According to a preferred embodiment the skincare agent is provided in a region, in the topsheet, partially overlapping the weakly-joined central region.

According to a preferred embodiment when an area of the weakly-joined central region is defined as S1 and an area of the region, in the topsheet, provided with the skincare agent and overlapping the weakly-joined central region is defined as S2, a value of S2/S1, which is a ratio of S2 to S1, is 0.3 or greater, preferably 0.4 or greater, and 1.0 or less, preferably 0.9 or less. More preferably, the area S1 of the weakly-joined central region is 300 cm² or greater, preferably 400 cm² or greater, and 700 cm² or less, preferably 600 cm² or less. The area S2 of the region in the topsheet may be provided with the skincare agent and overlapping the weakly-joined central region is 200 cm² or greater, preferably 250 cm² or greater, and 700 cm² or less, preferably 600 cm² or less.

According to a preferred embodiment when the area of the weakly-joined central region is defined as S1 and an area of the firmly-joined side region is defined as S3, a value of S3/S1, which is a ratio of S3 to S1, is 0.07 or greater, preferably 0.09 or greater, and 0.7 or less, preferably 0.6 or less. More preferably, the area S3 of the firmly-joined side region is preferably 50 cm² or greater, more preferably 60 cm² or greater, and preferably 200 cm² or less, more preferably 180 cm² or less.

According to a preferred embodiment the weakly-joined central region occupies a range from 40 to 85%, preferably from 45 to 80%, more preferably from 50 to 75%, with respect to an entire length W, in the width direction, of the topsheet, with a width-direction bisector being located in center, the bisector extending along the longitudinal direction and dividing the entire length, in the width direction, of the topsheet into two equal parts.

According to a preferred embodiment a length L1, in the longitudinal direction, of the weakly-joined central region is 40% or greater, preferably 50% or greater, and 100% or less, preferably 80% or less, with respect to an entire length L, in the longitudinal direction, of the topsheet. More preferably, the length L1, in the longitudinal direction, of the weakly-joined central region is 180 mm or greater, preferably 220 mm or greater, and 450 mm or less, preferably 400 mm or less.

According to a preferred embodiment a length W1, in the width direction, of the weakly-joined central region is 64 mm or greater, preferably 72 mm or greater, and 136 mm or less, preferably 128 mm or less.

According to a preferred embodiment a length W3, in the width direction, of the firmly-joined side region is 15% or greater, preferably 20% or greater, and 60% or less, preferably 55% or less, with respect to the entire length W, in the width direction, of the topsheet.

According to a preferred embodiment a difference in joining strength, when dry, between the weakly-joined central region and the firmly-joined side region is 0.5 N/50 mm or greater. More preferably, the difference in joining strength, when dry, between the weakly-joined central region and the firmly-joined side region is 0.5 N/50 mm or greater, preferably 0.7 N/50 mm or greater, and 2.3 N/50 mm or less, preferably 2.1 N/50 mm or less.

According to a preferred embodiment a hot-melt adhesive is applied to each of the weakly-joined central region and the firmly-joined side regions; and when a basis weight of the hot-melt adhesive in the weakly-joined central region is defined as B1 and a basis weight of the hot-melt adhesive in each of the firmly-joined side regions is defined as B2, a value of B2/B 1, which is a ratio of B2 to B1, is 1.2 or greater. More preferably, when the basis weight of the hot-melt adhesive in the weakly-joined central region is defined as B1 and the basis weight of the hot-melt adhesive in each of the firmly-joined side regions is defined as B2, the value of B2/B1, which is the ratio of B2 to B1, is preferably 1.2 or greater, more preferably 1.3 or greater, and preferably 2.5 or less, more preferably 2.4 or less.

According to a preferred embodiment a hot-melt adhesive is applied to the weakly-joined central region, and heat sealing is applied to the firmly-joined side regions.

According to a preferred embodiment the joining strength of the weakly-joined central region when dry is 1.0 N/50 mm or less. More preferably, the joining strength of the weakly-joined central region when dry is preferably 0.4 N/50 mm or greater, more preferably 0.5 N/50 mm or greater, and preferably 1.0 N/50 mm or less, more preferably 0.9 N/50 mm or less.

According to a preferred embodiment the joining strength of the weakly-joined central region when wet is 0.5 N/50 mm or less. More preferably, the joining strength of the weakly-joined central region when wet is preferably 0.1 N/50 mm or greater, more preferably 0.15 N/50 mm or greater, and preferably 0.5 N/50 mm or less, more preferably 0.45 N/50 mm or less.

According to a preferred embodiment a mass of the skincare agent per unit area of the weakly-joined central region is 0.00001 g/m² or greater, preferably 0.00005 g/m² or greater, more preferably 0.0001 g/m² or greater, and 0.05 g/m² or less, preferably 0.01 g/m² or less, more preferably 0.005 g/m² or less.

According to a preferred embodiment the topsheet is constituted by two or more layers of fiber layers, including a skin-side fiber layer to be arranged in a position closest to the wearer's skin, and a non-skin-side fiber layer to be arranged in a position farther from the wearer's skin than the skin-side fiber layer; and the skincare agent is provided to the skin-side fiber layer, wherein the skin-side fiber layer has a higher fiber density than the non-skin-side fiber layer, and is provided with the skincare agent. More preferably, the fiber density of the skin-side fiber layer is 1.25 times or greater, preferably 1.5 times or greater, and 8.0 times or less, preferably 7.0 times or less, the fiber density of the non-skin-side fiber layer. The fiber density of the skin-side fiber layer may be 250 pieces/mm² or greater, preferably 270 pieces/mm² or greater, and 400 pieces/mm² or less, preferably 380 pieces/mm² or less. Fineness of a constituent fiber of the skin-side fiber layer is smaller than that of the non-skin-side fiber layer. The fineness of the constituent fiber of the skin-side fiber layer may be 1.0 dtex or greater, preferably 1.3 dtex or greater, and 3.0 dtex or less, preferably 2.5 dtex or less.

According to a preferred embodiment MD bulk softness of the topsheet is 7.0 cN or less, preferably 6.5 cN or less, more preferably 6.0 cN or less, and 0.5 cN or greater. According to a preferred embodiment the absorbent article further comprises an absorbent core; the absorbent core includes a bending-guide portion extending in the longitudinal direction; and the bending-guide portion is in a position overlapping the weakly-joined central region in a planar view. More preferably, the absorbent core includes, as the bending-guide portions, a central bending-guide portion located in a central area in the width direction, and a pair of side bending-guide portions respectively located on both sides, in the width direction, of the central bending-guide portion; the central bending-guide portion overlaps the weakly-joined central region; and in a cross-sectional view along the width direction of the absorbent core, the absorbent core is configured to bend into a W-shape wherein the central bending-guide portion constitutes a mountain portion and the pair of side bending-guide portions constitutes valley portions. Further preferred is that a separation distance between one of the side bending-guide portions and the central bending-guide portion is the same as a separation distance between the other side bending-guide portion and the central bending-guide portion. The side bending-guide portions may be respectively provided in positions having symmetry with respect to a width-direction bisector that divides an entire length, in the width direction, of the absorbent core into two equal parts. A length, in the longitudinal direction, of the bending-guide portion may be 50 mm or greater, preferably 100 mm or greater, and 350 mm or less, preferably 300 mm or less, and from 50 to 350 mm, preferably from 100 to 300 mm. A width of the bending-guide portion may be 20 mm or less, preferably 15 mm or less, and 1 mm or greater, preferably 3 mm or greater.

According to a preferred embodiment the absorbent article further comprises an absorbent core; the absorbent article includes a front portion to be arranged on the wearer's front side, a crotch portion to be arranged in the wearer's crotch section, and a rear portion to be arranged on the wearer's rear side; the absorbent core includes a high-basis-weight region, and a low-basis-weight region having a relatively lower basis weight than the high-basis-weight region; and the low-basis-weight region is provided in the rear portion and is in a position overlapping the weakly-joined central region in a planar view. More preferably, a length, in the longitudinal direction, of the high-basis-weight region is 50% or greater, preferably 60% or greater, more preferably 70% or greater, and 90% or less, preferably 85% or less, more preferably 80% or less, and from 50 to 90%, preferably from 60 to 85%, more preferably from 70 to 80%, with respect to an entire length L4, in the longitudinal direction, of the absorbent core. A length L16, in the longitudinal direction, of the low-basis-weight region may be from 10 to 50%, preferably from 15 to 40%, more preferably from 20 to 30%, with respect to the entire length L4, in the longitudinal direction, of the absorbent core. The basis weight of the high-basis-weight region may be 120% or greater, preferably 140% or greater, and 250% or less, preferably 230% or less, and from 120 to 250%, preferably from 140 to 230%, with respect to the basis weight of the low-basis-weight region. The basis weight of the low-basis-weight region may be 100 g/m² or greater, preferably 150 g/m² or greater, more preferably 200 g/m² or greater, and 600 g/m² or less, preferably 550 g/m² or less, more preferably 500 g/m² or less, and from 100 to 600 g/m², preferably from 150 to 550 g/m², more preferably from 200 to 500 g/m².

According to a preferred embodiment the absorbent article includes a pair of leak-proof cuffs provided respectively to both lateral sides along the longitudinal direction on the skin-facing surface of the topsheet; the absorbent article includes an absorbent member and a backsheet as members in contact with the non-skin-facing surface of the topsheet; the weakly-joined central region joins a central area, in the width direction, of the topsheet and a central area, in the width direction, of the absorbent member; and each of the firmly joined side regions joins the topsheet to the absorbent member and to a region, in the backsheet, located near a base end section of the leak-proof cuff.

According to a preferred embodiment the absorbent article includes an absorbent member as the member in contact with the non-skin-facing surface of the topsheet; a sublayer sheet is provided between the topsheet and the absorbent member, the sublayer sheet being constituted by a nonwoven fabric; and the topsheet and the sublayer sheet are joined.

### Brief Description of Drawings

[Fig. 1] Fig. 1 is a plan view of a disposable diaper, which is an embodiment of an absorbent article of the present invention, as viewed from the skin-facing surface side, the disposable diaper being in a spread-open state where it has been spread in a planar state by stretching elastic members in various parts.
[Fig. 2] Fig. 2 is a cross-sectional view taken along line II-II of Fig. 1.
[Fig. 3] Fig. 3 is a plan view illustrating a positional relationship among a weakly-joined central region and firmly-joined side regions illustrated in Fig. 2, a topsheet, and an absorbent member.
[Fig. 4] Fig. 4 is a diagram for explaining an effect of the present invention, the diagram corresponding to Fig. 2.
[Fig. 5] Fig. 5 is a plan view illustrating major parts in another embodiment of an absorbent member according to the present invention, the diagram illustrating a positional relationship between an absorbent core of the absorbent member and a topsheet.
[Fig. 6] Fig. 6 is a diagram for explaining an effect of a disposable diaper including the absorbent core illustrated in Fig. 5, the diagram corresponding to Fig. 4.
[Fig. 7] Fig. 7 is a plan view illustrating major parts in yet another embodiment of an absorbent member according to the present invention, the diagram illustrating a positional relationship between an absorbent core of the absorbent member and a topsheet.

### Description of Embodiments

Skin trouble tends to occur in excretion sections, such as the anal area and genital area, and in peripheral sections thereof. The excretion sections are, however, located in depressed sections, like the groove between the buttocks (gluteal cleft) or gluteal folds, thereby making it difficult for a topsheet to contact those sections. This thus makes it difficult for a skincare agent provided to the topsheet to be transferred to the excretion sections and peripheral sections thereof. JP 2002-045391A and JP 2018-15520A do not disclose techniques for improving transferability of skincare agents to the excretion sections and peripheral sections thereof.

The present invention provides an absorbent article capable of overcoming the drawbacks of the aforementioned conventional art.

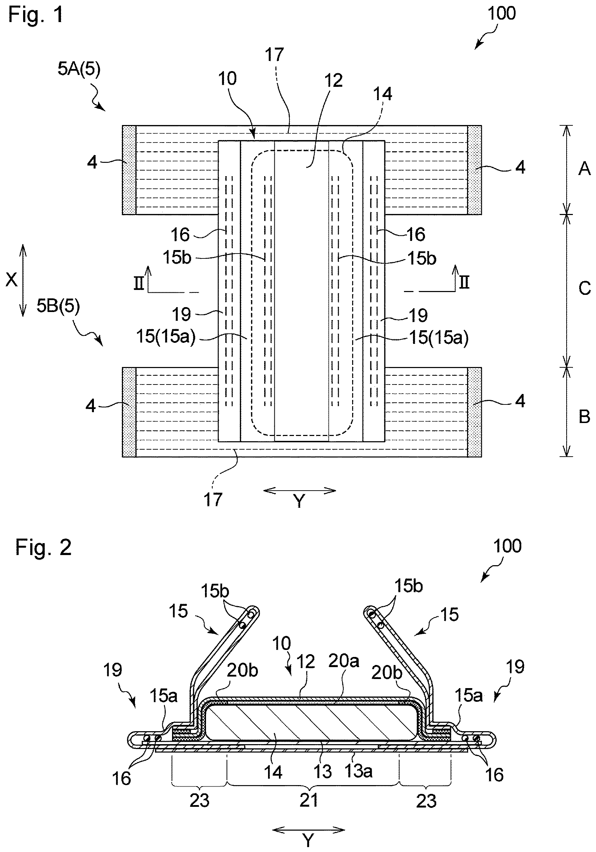

The present invention will be described below according to preferred embodiments thereof with reference to the drawings. Figs. 1 and 2 illustrate a fundamental structure of a disposable diaper 100, which is an embodiment of an absorbent article of the present invention. The diaper 100 has a longitudinally-oblong shape having: a longitudinal direction X corresponding to a direction extending from the wearer's front side to the rear side via the crotch section; and a width direction Y orthogonal to the longitudinal direction. The diaper 100 includes: a crotch portion C to be arranged in the wearer's crotch section; and a front portion A and a rear portion B respectively extending toward the front and rear from the crotch portion. The crotch portion C includes an excretion-section opposing region to be arranged in opposition to the wearer's excretion section when the diaper 100 is worn. The excretion-section opposing region is typically located in the diaper 100's central portion in the longitudinal direction X or in the vicinity thereof.

The diaper 100 is a pull-on type diaper having a waist opening through which the wearer's body passes, and a pair of leg openings through which the wearer's legs pass. In the diaper 100, both lateral side edge portions, which extend along the longitudinal direction X, of an outer cover 5 provided in both the front portion A and the rear portion B are joined together by a known joining means such as an adhesive, heat sealing, or ultrasonic sealing. In this way, the aforementioned waist opening and the pair of leg openings are formed.

Fig. 1 illustrates the diaper 100 in a spread-open and stretched state. The "spread-open and stretched state" of the diaper 100 refers to a state in which the joined lateral side edge portions, which extend along the longitudinal direction X, of the outer cover 5, which is provided in both the front portion A and the rear portion B, have been cut apart to bring the diaper in a spread-out (developed) state, and the elastic members in various parts of the spread-out diaper 100 are stretched so that the diaper is spread to its designed size (i.e., size when the diaper is spread out in a planar manner in a state where the effect of the elastic members is completely eliminated).

The diaper 100 includes an outer cover 5, and an absorbent assembly 10 fixed to the outer cover 5. The outer cover 5 includes a front panel 5A forming the front portion A, and a rear panel 5B forming the rear portion B. The front panel 5A and the rear panel 5B are joined together at a pair of side seals 4, 4. As illustrated in Fig. 1, the absorbent assembly 10 is fixed in a manner bridging the central portion, in the width direction Y, of the front panel 5A and the central portion, in the width direction Y, of the rear panel 5B. The sections of the absorbent assembly 10 respectively overlapping the front panel 5A and the rear panel 5B are joined respectively to the panels 5A, 5B either partially or over the entire surface by a known joining means such as an adhesive.

As illustrated in Fig. 2, the diaper 100 includes, as constituent members constituting the absorbent assembly 10: a topsheet 12 located closer to the surface facing the wearer's skin (i.e., skin-facing surface side); a backsheet 13 located on the non-skin-facing surface side; and an absorbent member 14 interposed between the two sheets 12, 13. In the Description, the "skin-facing surface" refers to a surface of the absorbent article, or a constituent member thereof (e.g., the topsheet 12), facing the wearer's skin side when the absorbent article is worn-i.e., on the side relatively closer to the wearer's skin. The "non-skin-facing surface" refers to a surface of the absorbent article, or a constituent member thereof, facing the opposite side from the skin (i.e., the clothing side) when the absorbent article is worn-i.e., on the side relatively farther from the wearer's skin. Herein, "when worn" refers to a state in which the absorbent article is maintained in its ordinary, proper wearing/attachment position, i.e., the correct wearing/attachment position of the absorbent article, and does not include cases where the absorbent article is deviated from the aforementioned wearing/attachment position.

For the topsheet 12, it is possible to use, for example, a liquid-permeable sheet, such as a nonwoven fabric or a perforated film. For the backsheet 13, it is possible to use, for example, a sparingly liquid-permeable film or a spunbond-meltblown-spunbond layered nonwoven fabric. A multitude of fine pores may be provided in the sparingly liquid-permeable film, to make the film vapor-permeable. In the present embodiment, with the aim of further improving, for example, the texture of the diaper, an outermost sheet 13a having excellent texture, such as a nonwoven fabric, is layered on the outer surface of the backsheet 13.

The absorbent member 14 includes an absorbent core (not illustrated). The absorbent core may be constituted by, for example: a fiber stack of hydrophilic fibers such as cellulose, e.g., pulp; a mixed fiber stack including hydrophilic fibers and an absorbent polymer; an accumulation of an absorbent polymer; or a layered structure wherein an absorbent polymer is supported between two absorbent sheets. The absorbent member 14 may or may not include a core-wrap sheet that covers the absorbent core. In cases of providing a core-wrap sheet, it will suffice if at least the skin-facing surface of the absorbent core is covered by a liquid-permeable core-wrap sheet. Alternatively, the entire surface, including the skin-facing surface and the non-skin-facing surface, may be covered by the core-wrap sheet. For the core-wrap sheet, it is possible to use, for example, tracing paper (tissue paper) made from a hydrophilic fiber, or a liquid-permeable nonwoven fabric.

The diaper 100 includes a pair of leak-proof cuffs 15, 15 provided respectively to both lateral sides along the longitudinal direction X on the skin-facing surface of the topsheet 12, the leak-proof cuffs extending along the longitudinal direction X. Each leak-proof cuff 15 includes a base end section and a free end. Each leak-proof cuff 15 has its base end section on the skin-facing surface side of the diaper 100, and stands up from the skin-facing surface side. Each leak-proof cuff 15 includes: a cuff-forming sheet 15a that is continuous over the entire length, in the longitudinal direction X, of the absorbent member 14; and elastic members 15b, such as rubber threads, provided in a stretched state to the leak-proof cuff 15's free end or in the vicinity thereof. One lateral side edge portion, in the width direction, of the cuff-forming sheet 15a is located at the base end section of the leak-proof cuff 15, whereas the other lateral side edge portion is located between the backsheet 13 and the outermost sheet 13a. In a state where the diaper 100 is worn, the elastic members 15b contract, and thereby, the leak-proof cuffs 15 stand up toward the wearer's body. Thus, liquid excreted onto the topsheet 12 is effectively prevented from spreading on the topsheet 12 and leaking outward in the width direction of the diaper 100. For the cuff-forming sheet 15a, it is possible to use, for example, an air-permeable material having liquid resistance or water repellency.

In the crotch portion C of the diaper 100, a portion of each cuff-forming sheet 15a located more outside, in the width direction Y, than the standing section of the leak-proof cuff 15 forms a leg peripheral edge portion 19 to be arranged around the wearer's leg. Leg-portion elastic members 16 are provided in a stretched state to each leg peripheral edge portion 19, and the contraction thereof forms leg gathers in the crotch portion C for improving fittability around the wearer's leg when the diaper is worn.

The front panel 5A and the rear panel 5B constituting the outer cover 5 each include: an outer sheet constituting the outer surface of the diaper; an inner sheet provided on the inner surface side of the outer sheet; and a plurality of thread-shaped elastic members 17 provided in a stretched state between the two sheets. The contraction of the elastic members 17 improves fittability to sections around the wearer's hip and waist.

As illustrated in Fig. 2, the topsheet 12 is joined, on the non-skin-facing surface thereof, to a member in contact with the non-skin-facing surface. More specifically, the topsheet 12 is in contact with the absorbent member 14. Also, the topsheet 12 is in contact with the backsheet 13 in a section more toward the outer side, in the width direction Y, than the absorbent member 14. The topsheet 12 is joined to the members in contact with the non-skin-facing surface of the topsheet 12 by a known joining means. Examples of known joining means include various adhesives such as hot-melt adhesives, and fusion-bond means such as heat sealing, ultrasonic sealing and high-frequency sealing.

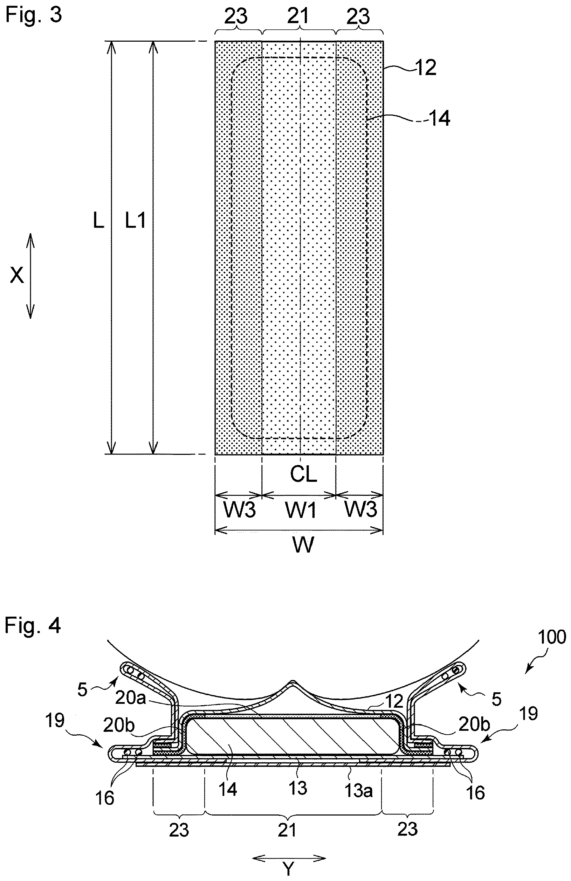

As illustrated in Figs. 2 and 3, a region, within the topsheet 12's non-skin-facing surface, that is joined to the other member includes a weakly-joined central region 21 and a pair of firmly-joined side regions 23 sandwiching the weakly-joined central region 21. The weakly-joined central region 21 is a joined region located in a central area, in the width direction, of the topsheet 12, and having a relatively low joining strength. The firmly-joined side regions 23 are joined regions respectively located in side areas, in the width direction, of the topsheet 12 and adjacent to the weakly-joined central region 21, and having a relatively high joining strength. The weakly-joined central region 21 and the firmly-joined side regions 23 all extend along the longitudinal direction of the diaper 100. In the present embodiment, as illustrated in Fig. 2, the weakly-joined central region 21 joins a central area, in the width direction, of the topsheet 12 and a central area, in the width direction, of the absorbent member 14. On the other hand, each of the firmly-joined side regions 23 joins a side area, in the width direction, of the topsheet 12 to a side area, in the width direction, of the absorbent member 14 and to a region, in the backsheet 13, located near the base end section of the leak-proof cuff 15.

The topsheet 12 is provided with a skincare agent. The skincare agent has such effects as to protect and/or heal the wearer's skin. By providing a skincare agent to the topsheet 12, the skincare agent transfers from the topsheet 12 to the wearer's skin in a state where the diaper 100 is worn, thereby achieving a skincare effect. According to the present embodiment, the skincare agent is transferred smoothly as will be described further below, thereby further effectively achieving the skincare effect. Examples of the skincare agent may include: various plant extracts; moisturizers, such as collagen, natural moisturizing factors (NMF), 1,3-butylene glycol, propylene glycol, dipropylene glycol, and glycerin; stratum corneum softeners, such as arginine and guanidine derivatives (e.g., organic acid salts (e.g., succinate, glycolate, lactate, malate, citrate, tartrate, etc.) of 2-(2-hydroxyethoxy)ethyl guanidine); anti-inflammatory agents, such as guaiazulen, tannin, and gallic acid derivatives; and components used as emollient agents in the field of cosmetics.

Concrete examples of plant extracts may include oat extract, seaweed (sea oak) extract, citron extract, hamamelis (witch hazel) extract, burnet extract, asunaro (hiba) extract, aloe extract, phellodendron bark extract, field horsetail extract, chamomile extract, eucalyptus extract, and peach leaf extract. From the viewpoint of achieving excellent skincare effects, it is preferable to use citron extract, hamamelis extract, asunaro extract, aloe extract, phellodendron bark extract, eucalyptus extract, or peach leaf extract, and more preferably hamamelis extract.

For example, a plant extract can be obtained by: drying and pulverizing, or pulverizing without drying, one of various plants in its entirety, or at least one part thereof among the leaves, bark, root, or branches; and then subjecting the pulverized product to extraction in a solvent at atmospheric temperature or under heat, or with an extracting instrument such as a Soxhlet extractor. It is preferable to use a 1,3-butylene glycol extract as the plant extract from the viewpoint that explosion-proofing measures are unnecessary even when heating is performed during the manufacturing process, and also from the viewpoint that degradation in absorbency can be suppressed even when applied to the topsheet 12.

In general, a plant extract exists in a state containing not only active ingredients, but also the solvent component used for extraction as well as moisture. It will suffice if the amount of active ingredients in the plant extract in this state (excluding volatile solvent components and moisture) is about 0.001 to 5 mass%. In cases where 1,3-butylene glycol is used as the solvent component for extraction, it will suffice if the total amount of the active ingredients and 1,3-butylene glycol in the plant extract is within the aforementioned range. The amount of active ingredients in the plant extract is preferably about 0.001 to 10 mass% with respect to the amount of the solvent component.

Concrete examples of skincare agents used as emollient agents may include liquid paraffin, silicone oil, animal and vegetable oils (olive oil, jojoba oil, safflower oil, squalene, squalene, etc.), monoglycerides, diglycerides, triglycerides, aliphatic ethers (myristyl-1,3-dimethylbutyl ether, palmityl-1,3-dimethylbutyl ether, stearyl-1,3-dimethylbutyl ether, palmityl-1,3-methylpropyl ether, stearyl-1,3-methylpropyl ether, etc.), isostearyl-cholesterol ester, paraffin wax, C₁₂-C₂₂ fatty acids, C₁₂-C₄₄ fatty acid ethers, C₁₂-C₂₂ fatty alcohols, vaseline, sorbitan fatty acid esters (any of monoesters, diesters, or triesters), polyoxyethylene sorbitan fatty acid esters (any of monoesters, diesters, or triesters), metal soaps (magnesium stearate, etc.), sucrose fatty acid esters, cyclodextrin fatty acid esters, silicone, silicone-based resins, and emollient agents, or lotion compositions containing an emollient agent and an immobilizing agent, as used in the inventions disclosed in JP2004-255164A and JP2012-143543A.

One type of the aforementioned hydrophobic skincare agent may be used singly, or two or more types may be used in combination.

As described above, the topsheet 12 is joined to other members in the weakly-joined central region 21 and the firmly-joined side regions 23. Therefore, in cases where abrasion force is applied to the topsheet 12 in a state where the diaper 100 is worn, or in cases where the topsheet 12 gets wet by body fluid, a section of the topsheet 12 corresponding to the weakly-joined central region 21 peels off from the absorbent member 14, which is the member joined to the topsheet 12. On the other hand, in sections of the topsheet 12 corresponding to the firmly-joined side regions 23, the topsheet 12 is kept joined to the members in contact with the topsheet's non-skin-facing surface. As a result, the central area, in the width direction, of the topsheet 12 separates from the absorbent member 14 and comes into sufficient contact with the wearer's skin. Particularly, as illustrated in Fig. 4, the central area, in the width direction, of the topsheet 12 enters the depressed sections, such as the gluteal cleft and the gluteal folds, along with the wearer's movement, and thus comes into sufficient contact with the excretion sections, such as the anal area and genital area, and peripheral sections thereof. As a result, the skincare agent provided to the topsheet 12 can effectively be transferred to the excretion sections and peripheral sections thereof.

From the viewpoint of enabling the topsheet 12 to enter the depressed sections of the buttocks more easily, it is preferable that the weakly-joined central region 21 is located between the pair of leak-proof cuffs 15, 15. In this case, the weakly-joined central region 21 may be present not only between the pair of leak-proof cuffs 15, 15, but may also extend more outward, in the width direction Y, than the respective free ends of the leak-proof cuffs 15.

From the same viewpoint, it is preferable that, when worn, the weakly-joined central region 21 is located in opposition to both the wearer's urinary excretion section and fecal excretion section. In this case, the weakly-joined central region 21 is located in the excretion-section opposing region of the crotch portion C.

In the diaper 100, it is advantageous to apply the skincare agent to the skin-facing surface of the topsheet 12. From the viewpoint of enabling the skincare agent to be transferred to the skin easily, it is preferable that the topsheet 12 is provided with the skincare agent in a region overlapping the weakly-joined central region 21. In this case, the skincare agent may be provided in a region, in the topsheet 12, partially overlapping the weakly-joined central region 21, or may be provided in a region, in the topsheet 12, overlapping the entire weakly-joined central region 21. The skincare agent may be, for example, provided to a constituent fiber of the topsheet 12. More specifically, the skincare agent may be applied to the surface of the fiber, or may be kneaded into a resin constituting the fiber.

From the viewpoint of improving transferability of the skincare agent, it is preferable that, when the area of the weakly-joined central region 21 is defined as S1 and the area of the region, in the topsheet 12, provided with the skincare agent and overlapping the weakly-joined central region 21 is defined as S2, the value of S2/S1, which is the ratio of S2 to S1, is preferably 0.3 or greater, more preferably 0.4 or greater, and preferably 1.0 or less, more preferably 0.9 or less, and preferably from 0.3 to 1.0, more preferably from 0.4 to 0.9. Hereinbelow, the region, in the topsheet 12, provided with the skincare agent and overlapping the weakly-joined central region 21 is also referred to as "skincare agent region".

From the same viewpoint, the area S1 of the weakly-joined central region 21 is preferably 300 cm² or greater, more preferably 400 cm² or greater, and preferably 700 cm² or less, more preferably 600 cm² or less, and preferably from 300 to 700 cm², more preferably from 400 to 600 cm².

From the same viewpoint, the area S2 of the skincare agent region is preferably 200 cm² or greater, more preferably 250 cm² or greater, and preferably 700 cm² or less, more preferably 600 cm² or less, and preferably from 200 to 700 cm², more preferably from 250 to 600 cm².

From the viewpoint of achieving shape retainability of the absorbent assembly 10 more reliably when the central area, in the width direction, of the topsheet 12 peels off, it is preferable that, when the area of the weakly-joined central region 21 is defined as S1 and the area of the firmly-joined side region 23 is defined as S3, the value of S3/S1, which is the ratio of S3 to S1, is preferably 0.07 or greater, more preferably 0.09 or greater, and preferably 0.7 or less, more preferably 0.6 or less, and preferably from 0.07 to 0.7, more preferably from 0.09 to 0.6.

From the same viewpoint, the area S3 of the firmly-joined side region 23 is preferably 50 cm² or greater, more preferably 60 cm² or greater, and preferably 200 cm² or less, more preferably 180 cm² or less, and preferably from 50 to 200 cm², more preferably from 60 to 180 cm².

The respective ranges of the weakly-joined central region 21 and the firmly-joined side regions 23 can be identified by the joining strengths measured according to the "Method for Measuring Joining Strength" described below.

The skincare agent region in the weakly-joined central region 21 can be identified by verifying the change in water contact angle of the topsheet 12. The water contact angle can be measured, for example, according to the method disclosed in JP2015-142721A. For example, in a skincare agent region in which a water-soluble, water-dispersible skincare agent has been applied, the contact angle between water and the topsheet's constituent fiber becomes less than 90 degrees; whereas in a skincare agent region in which a non-water-soluble, non-water-dispersible skincare agent has been applied, the contact angle between water and the topsheet's constituent fiber becomes equal to or greater than 90 degrees. Whether or not the skincare agent is water-soluble and water-dispersible is determined according to the following method. A sheet having a skincare agent applied thereto is immersed in water (liquid temperature: 25°C), the amount of water being 10 times the mass of the sheet; if, after being left for 24 hours, the skincare agent does not completely dissolve and does not disperse, the skincare agent is determined as being water-soluble and water-dispersible. On the other hand, if, after being left for 24 hours, the skincare agent completely dissolves or disperses, the skincare agent is determined as being not water-soluble or water-dispersible.

From the viewpoint of facilitating contact between the excretion section and the topsheet 12, it is preferable that the dimensions of the weakly-joined central region 21 and the firmly-joined side regions 23 are within the following ranges.

The weakly-joined central region 21 occupies a range preferably from 40 to 85%, more preferably from 45 to 80%, even more preferably from 50 to 75%, with respect to the entire length W, in the width direction, of the topsheet 12, with a width-direction bisector CL being located in center. The width-direction bisector CL is a line extending along the longitudinal direction X and dividing the entire length, in the width direction Y, of the topsheet 12 into two equal parts (see Fig. 3).

The length L1 (see Fig. 3), in the longitudinal direction X, of the weakly-joined central region 21 is preferably 40% or greater, more preferably 50% or greater, and preferably 100% or less, more preferably 80% or less, and preferably from 40 to 100%, more preferably from 50 to 80%, with respect to the entire length L (see Fig. 3), in the longitudinal direction X, of the topsheet 12.

The length W1 (see Fig. 3), in the width direction Y, of the weakly-joined central region 21 is preferably 64 mm or greater, more preferably 72 mm or greater, and preferably 136 mm or less, more preferably 128 mm or less, and preferably from 64 to 136 mm, more preferably from 72 to 128 mm.

The length L1 (see Fig. 3), in the longitudinal direction X, of the weakly-joined central region 21 is preferably 180 mm or greater, more preferably 220 mm or greater, and preferably 450 mm or less, more preferably 400 mm or less, and preferably from 180 to 450 mm, more preferably from 220 to 400 mm.

The length W3 (see Fig. 3), in the width direction Y, of the firmly-joined side region 23 is preferably 15% or greater, more preferably 20% or greater, and preferably 60% or less, more preferably 55% or less, and preferably from 15 to 60%, more preferably from 20 to 55%, with respect to the entire length W (see Fig. 3), in the width direction Y, of the topsheet 12.

The length, in the longitudinal direction X, of the firmly-joined side region 23 is preferably within the preferred range for the length L1, in the longitudinal direction X, of the weakly-joined central region 21.

From the viewpoint of further facilitating peeling of the central area, in the width direction, of the topsheet 12, it is preferable that, in the weakly-joined central region 21, the topsheet 12 is peelable from the member in contact with the topsheet 12 according to a crease-flex test evaluation described below, and it is also preferable that, in the firmly-joined side regions 23, the topsheet 12 is not peelable from the member in contact with the topsheet 12 according to abrasion under the same conditions.

The crease-flex test evaluation is performed as follows.

In a diaper, 250 g of physiological saline solution is injected to the central area, in the width direction, of the topsheet, and then the diaper is left standing for 5 minutes. Next, a fastening tape is cut out from a Merries (registered trademark) diaper from Kao Corporation; an attachment portion of the fastening tape is attached to the topsheet, and then, a 1-kg weight is placed thereon and is left standing for 10 seconds. Then, the fastening tape is stripped off from the topsheet, and peeling between the topsheet and the member in contact with the topsheet is visually observed. The aforementioned operation is repeated 5 times; in cases where the aforementioned peeling is observed at least 4 times, it is determined that the topsheet and a member in contact with the topsheet are peelable.

From the same viewpoint, a difference in joining strength between the weakly-joined central region 21 and the firmly-joined side region 23 is preferably 0.5 N/50 mm or greater, more preferably 0.7 N/50 mm or greater, and preferably 2.3 N/50 mm or less, more preferably 2.1 N/50 mm or less, and preferably from 0.5 to 2.3 N/50 mm, more preferably from 0.7 to 2.1 N/50 mm. The joining strength is measured according to the method described below. The respective joining strengths of the weakly-joined central region 21 and the firmly-joined side regions 23 can be made different from one another by, for example: using joining means having different joining strengths for the respective regions 21, 23; or using the same type of adhesive for the respective regions 21, 23, but varying the basis weight of the adhesive.

### Method for Measuring Joining Strength:

In an environment of 22°C, 65% RH, a disposable diaper is stretched until the gathers formed by contraction of the various elastic members disappear. Then, a section where the topsheet and the entire region of the absorbent member overlap one another in the thickness direction, as viewed in a planar view, is cut out and employed as a measurement sample. Next, the measurement sample is mounted to a tensile tester (e.g., a Tensilon tensile tester RTA-100 from Orientec Co., Ltd.) in a manner such that the topsheet will be peeled along the diaper's width direction in the measurement piece. For example, one end, in the width direction, of the topsheet is attached to the upper chuck of the tensile tester, and the absorbent member is attached to the lower chuck. The distance between the upper and lower chucks is set to 10 mm. Next, with the position of the lower chuck fixed, the upper chuck is raised at a rate of 300 mm/min, to peel the topsheet along the diaper's width direction. During this process, the tensile load that changes along with the elevation of the upper chuck-i.e., along with the pulling distance-is measured. In a pulling distance/tensile load curve obtained by this measurement, with the increase in the pulling distance, a relatively high tensile load appears continuously after the first maximum point, and then, a relatively low tensile load appears continuously, and thereafter, a relatively high tensile load continuously appears again. That is, the tensile load changes in three stages. In this pulling distance/tensile load curve, the first stage is defined as the range that starts from the first maximum point initially appearing after starting the measurement, and during which a tensile load of substantially the same level as the first maximum point is observed. The second stage is defined as the range after the first stage and during which a tensile load relatively smaller than that of the first stage is observed. The third stage is defined as the range after the second stage and during which a tensile load relatively larger than that of the second stage is observed. The joining strength of one of the firmly-joined side regions is the mean value of the integral of the tensile load within a range, in the first stage, from the first maximum point to 10 mm toward the positive side therefrom. The joining strength of the weakly-joined central region is the mean value of the integral of the tensile load within a range from a point indicating the first-appearing maximum in the second stage to 10 mm toward the positive side therefrom. The joining strength of the other firmly-joined side region is the mean value of the integral of the tensile load within a range from a point indicating the first-appearing maximum in the third stage to 10 mm toward the positive side therefrom. The joining strength is measured using a measurement piece in a dry state, unless specifically mentioned otherwise. The "joining strength when dry" described further below refers to the joining strength of a measurement piece in a dry state, and the "joining strength when wet" described further below refers to the joining strength of a measurement piece in a wet state. "Wet state" refers to a state where the entire measurement sample has been immersed in water.

In the present embodiment, the respective joining strengths of the weakly-joined central region 21 and the firmly-joined side regions 23 can be adjusted by applying hot-melt adhesives 20a, 20b respectively to the weakly-joined central region 21 and the firmly-joined side regions 23 (see Fig. 2). For example, when the basis weight of the hot-melt adhesive 20a in the weakly-joined central region 21 is defined as B1 and the basis weight of the hot-melt adhesive 20b in each of the firmly-joined side regions 23 is defined as B2, the value of B2/B1, which is the ratio of B2 to B1, is preferably 1.2 or greater, more preferably 1.3 or greater, and preferably 2.5 or less, more preferably 2.4 or less, and preferably from 1.2 to 2.5, more preferably from 1.3 to 2.4. In this way, the joining strength of the firmly-joined side regions 23 can be made higher than that of the weakly-joined central region 21.

Instead of applying hot-melt adhesives to the weakly-joined central region 21 and the firmly-joined side regions 23, a hot-melt adhesive may be applied to the weakly-joined central region 21, and heat sealing may be applied to the firmly-joined side regions 23.

From the viewpoint of further increasing contact of the topsheet 12 to the excretion section and further improving the skincare effect when the diaper is wetted by excrement, it is preferable that, in the weakly-joined central region 21, the joining strength when wet is lower than the joining strength when dry. In this case, the joining strength of the weakly-joined central region 21 when dry is preferably 0.4 N/50 mm or greater, more preferably 0.5 N/50 mm or greater, and preferably 1.0 N/50 mm or less, more preferably 0.9 N/50 mm or less, and preferably from 0.4 to 1.0 N/50 mm, more preferably from 0.5 to 0.9 N/50 mm. Further, the joining strength of the weakly-joined central region 21 when wet is preferably 0.1 N/50 mm or greater, more preferably 0.15 N/50 mm or greater, and preferably 0.5 N/50 mm or less, more preferably 0.45 N/50 mm or less, and preferably from 0.1 to 0.5 N/50 mm, more preferably from 0.15 to 0.45 N/50 mm.

In cases of varying the joining strength of the weakly-joined central region 21 between its dry state and its wet state, it is possible to use, for example, a type of adhesive whose joining strength changes between when wet and when dry. As such an adhesive, it is possible to use, as appropriate, a type of adhesive whose joining strengths fall within the aforementioned ranges.

From the viewpoint of transferring the skincare agent to the skin more easily, the mass of the skincare agent per unit area of the weakly-joined central region 21 is preferably 0.00001 g/m² or greater, more preferably 0.00005 g/m² or greater, even more preferably 0.0001 g/m² or greater, and preferably 0.05 g/m² or less, more preferably 0.01 g/m² or less, even more preferably 0.005 g/m² or less, and preferably from 0.00001 to 0.05 g/m², more preferably from 0.00005 to 0.01 g/m², even more preferably from 0.0001 to 0.005 g/m². The mass of the skincare agent per unit area can be found according to the following method.

### Method for Measuring Mass of Skincare Agent per Unit Area:

A measurement piece having an area of at least 300 mm² is cut out from a section of the topsheet overlapping the weakly-joined central region which has been identified by the aforementioned "Method for Measuring Joining Strength". If the area cut out from one section does not amount to 300 mm², then measurement pieces are cut out from a plurality of sections, so that the total area becomes at least 300 mm². Next, the measurement piece is subjected to a multistage solvent extraction method using solvents ranging from non-polar solvents to polar solvents, to isolate the skincare agent present in the measurement piece and thereby obtain a solution containing a single composition. The obtained solution is dried and solidified, and the chemical structure of the skincare agent is identified by using, in combination, ¹H-NMR (nuclear magnetic resonance spectroscopy), IR (infrared spectroscopy), LC (liquid chromatography), GC (gas chromatography), MS (mass spectrometry), GPC (gel permeation chromatography), fluorescent X-rays, etc. Then, a calibration curve is created by known analytic means, such as GC and LC, by using an agent having the identified chemical structure as a standard reference material, and based thereon, the amount of skincare agent in the measurement piece is determined. Based on this quantification result, the mass of the skincare agent per unit area (1 mm²) is determined. In cases where there are a plurality of types of skincare agents in the measurement piece, the mass of each of the skincare agents is summed, to find the total mass of skincare agents per unit area (1 mm²), and this is considered the "mass of the skincare agent per unit area".

The topsheet 12 may have a single-layer structure, or have a layered structure including two or more layers of fiber layers. In cases where the topsheet 12 has a layered structure, the fiber layers in the layered structure include: a skin-side fiber layer to be arranged in a position closest to the wearer's skin; and a non-skin-side fiber layer to be arranged in a position farther from the wearer's skin than the skin-side fiber layer. In cases where the topsheet 12 has a layered structure including three or more layers of fiber layers, the topsheet 12 includes a single skin-side fiber layer forming the skin-facing surface, and two or more non-skin-side fiber layers.

From the viewpoint of improving the transferability of the skincare agent to the skin, it is preferable that the skincare agent is provided on the skin-facing surface side of the topsheet 12. In cases where the topsheet 12 has a layered structure, it is preferable that the skincare agent is provided to the skin-side fiber layer.

In cases where the topsheet 12 has a layered structure, it is preferable that the skin-side fiber layer has a higher fiber density than the non-skin-side fiber layer, and is provided with the skincare agent. With this configuration, the skincare agent can easily be retained in the skin-side fiber layer by capillary action, and thus, the transferability of the skincare agent to the skin can be improved. From the viewpoint of achieving this effect more reliably, it is preferable that the respective fiber densities of the skin-side fiber layer and the non-skin-side fiber layer are within the following ranges. It is preferable that the fiber density of the skin-side fiber layer is preferably 1.25 times or greater, more preferably 1.5 times or greater, and preferably 8.0 times or less, more preferably 7.0 times or less, and preferably from 1.25 to 8.0 times, more preferably from 1.5 to 7.0 times, the fiber density of the non-skin-side fiber layer. The fiber density of the skin-side fiber layer is preferably 250 pieces/mm² or greater, more preferably 270 pieces/mm² or greater, and preferably 400 pieces/mm² or less, more preferably 380 pieces/mm² or less, and preferably from 250 to 400 pieces/mm², more preferably from 270 to 380 pieces/mm². The fiber density of the non-skin-side fiber layer is preferably 50 pieces/mm² or greater, more preferably 70 pieces/mm² or greater, and preferably 200 pieces/mm² or less, more preferably 180 pieces/mm² or less, and preferably from 50 to 200 pieces/mm², more preferably from 70 to 180 pieces/mm². The fiber density of each fiber layer can be found according to the following method.

### Method for Measuring Fiber Density:

A cut cross section of a topsheet 12 is observed under magnification with a scanning electron microscope, with the magnification adjusted so that cross-sectional surfaces of about 30 to 60 pieces of fibers can be counted (150x to 500x). In the visual field of observation of the cut cross section, the area occupied by the fibers per given visual field area (0.02 mm²) is measured by image analysis. More specifically, by using image processing software such as ImageJ, the obtained image is binarized in terms of luminosity by setting the threshold at the luminosity boundary between fibers and sections without fibers. Generally speaking, when binarized into black and white, the fibers become white and the sections without fibers become black; thus, it is possible to identify, in the thickness direction of the measurement object (topsheet), the region with the most white sections as the skin-side fiber layer, and identify the region with more black sections than the skin-side fiber layer as the non-skin-side fiber layer. Next, the number of cross-sectional surfaces of cut fibers is counted within the aforementioned given area (about 0.02 mm²), and this is converted into the number of cross-sectional surfaces of fibers per 1 mm² (pieces/mm²). This measurement is performed at three discretionary locations in the topsheet, and the average value is considered the fiber density of the object. For the scanning electron microscope, it is possible to use, for example, JCM-5100 (trade name) from JEOL Ltd.

In cases where the topsheet 12 has a layered structure, it is preferable that the fineness of a constituent fiber of the skin-side fiber layer is smaller than that of the non-skin-side fiber layer. With this configuration, the fineness of the constituent fiber of the non-skin-side fiber layer becomes relatively large, which, in turn, reduces the joining area between the topsheet 12's non-skin-facing surface and the member joined to the topsheet 12, thereby making the topsheet 12 more easily peelable in the weakly-joined central region 21. Further, since the fineness of the constituent fiber of the skin-side fiber layer is relatively small, the topsheet 12, which has peeled off, is less likely to damage the skin when it enters the wearer's excretion section. From the viewpoint of achieving these effects more reliably, it is preferable that the respective fineness of constituent fibers of the skin-side fiber layer and the non-skin-side fiber layer is within the following ranges. The fineness of the constituent fiber of the skin-side fiber layer is preferably 1.0 dtex or greater, more preferably 1.3 dtex or greater, and preferably 3.0 dtex or less, more preferably 2.5 dtex or less, and preferably from 1.0 to 3.0 dtex, more preferably from 1.3 to 2.5 dtex. The fineness of the constituent fiber of the non-skin-side fiber layer is preferably 3.3 dtex or greater, more preferably 4.0 dtex or greater, and preferably 10.0 dtex or less, more preferably 7.0 dtex or less, and preferably from 3.3 to 10.0 dtex, more preferably from 4.0 to 7.0 dtex. In cases where the topsheet 12 includes a plurality of non-skin-side fiber layers, it is preferable that the fineness of the constituent fiber of each non-skin-side fiber layer is within the aforementioned range. The fineness of fiber is measured as follows.

### Method for Measuring Fineness of Fiber:

A measurement sample is prepared by cutting out the topsheet in a rectangular shape that is 50 mm wide and 100 mm long (area: 5000 mm²). Then, in a cross-sectional view of the measurement sample, an electron microscope is used to measure each thickness of 10 pieces of standard fibers located at a position separated by 0.2 mm, in the thickness direction, from the skin-facing surface of the measurement sample, to calculate the average fiber thickness Dn (µm). Then, a resin constituting the standard fibers located at a position separated by 0.2 mm, in the thickness direction, from the skin-facing surface is specified, and a differential scanning calorimeter (DSC) is used to find the theoretical fiber presence density Pn (g/cm³). From the obtained average fiber thickness Dn (µm) and theoretical fiber presence density Pn (g/cm³), the weight (g) per fiber length of 10,000 m is calculated, and the calculated value is considered the fineness (dtex) of the fiber constituting the skin-side fiber layer. The fineness of fiber constituting the non-skin-side fiber layer is measured according to the same method as for the fineness of the fiber constituting the skin-side fiber layer, except that the target of measurement is 10 pieces of standard fibers located at a position separated by 0.2 mm, in the thickness direction, from the non-skin-facing surface of the measurement sample, in a cross-sectional view of the measurement sample.

A topsheet 12 having fiber densities and/or constituent fiber fineness differing between the skin-side and non-skin-side fiber layers can be manufactured by selecting, as appropriate, the thickness and/or the fiber resin material of the constituent fiber for each fiber layer. Alternatively, a topsheet having a relatively high fiber density on the skin-side fiber layer side can be manufactured by heating and compressing a web, which becomes the skin-side fiber layer, in a step for forming the fiber layers.

The shape of the surface of the topsheet 12 may take any one of various forms known in the present technical field without particular limitation. For example, both the skin-facing surface and the non-skin-facing surface of the topsheet 12 may be flat, but it is preferable that one, or both, of the skin-facing surface and the non-skin-facing surface have a projecting-and-depressed shape. Providing the non-skin-facing surface side of the topsheet with a projecting-and-depressed shape can reduce the joining area between the topsheet 12 and the member joined to the topsheet 12 in the weakly joined central region 21, thereby making the topsheet 12 more easily peelable. On the other hand, providing the skin-facing surface side of the topsheet with a projecting-and-depressed shape reduces the contact area between the topsheet 12, which has peeled off, and the excretion section, thereby making the topsheet 12 less likely to damage the skin when it enters the wearer's excretion section. Examples of topsheets having a projecting-and-depressed shape include sheets having, on one or both of the topsheet's skin-facing surface and non-skin-facing surface: a multitude of projections formed in a scattered manner; or ridges and grooves formed alternately and extending in a single direction. The projections and ridges may have a hollow interior or a solid interior. Topsheets having a projecting-and-depressed shape can be manufactured according to, for example, manufacturing methods disclosed in JP2015-112343A and JP2016-117977A.

From the viewpoint of suppressing the topsheet 12, which has peeled off from the absorbent member 14, from causing an unnatural feel when it enters the wearer's excretion section, it is preferable that the MD bulk softness of the topsheet 12 is preferably 7.0 cN or less, more preferably 6.5 cN or less, even more preferably 6.0 cN or less. As regards the lower limit of the MD bulk softness, the closer it is to zero, the more preferable; but from the viewpoint of stably manufacturing the diaper, the MD bulk softness is preferably 0.5 cN or greater. The MD bulk softness is measured according to the following measurement method.

### Method for Measuring MD Bulk Softness:

To measure the MD bulk softness, a section that is 150 mm long in the CD direction, which is along the direction of orientation of the topsheet's constituent fiber, and 30 mm wide in the MD direction, which is orthogonal to the CD direction, is cut out from the topsheet in an environment of 22°C, 65% RH, and this is employed as a measurement piece. The ends of this measurement piece are stapled with a stapler at two upper and lower sites so as to form a 45-mm-dia. ring. The staples of the stapler are oriented so that they are long in the CD direction. Next, a tensile tester (e.g., a Tensilon tensile tester RTA-100 from Orientec Co., Ltd.) is used, and the ring-shaped measurement piece is placed on the sample stage so as to stand up like a cylinder, and is compressed from above with a flat plate arranged substantially parallel to the stage at a compression rate of 10 mm/minute, and the maximum load during this compression is measured. This measurement is performed for three discretionary sections cut out from the topsheet, and the average value is considered the MD bulk softness.

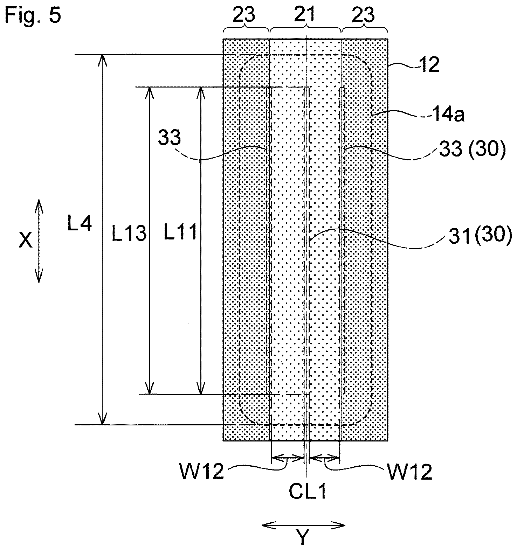

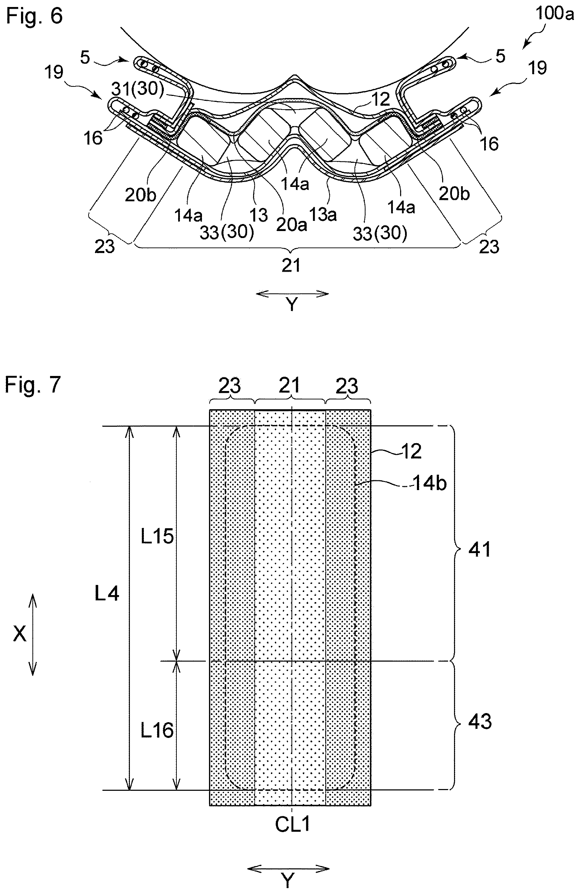

Next, other embodiments of the absorbent article of the present invention will be described with reference to the drawings. In these other embodiments, the explanation given in the foregoing embodiment applies as appropriate, unless there are contradictions. In Figs. 5 to 7, features identical to the foregoing embodiment are accompanied by the same reference signs as in the foregoing embodiment. In diaper 100a illustrated in Fig. 6, the core-wrap sheet covering the absorbent core 14a is omitted from illustration for the sake of facilitating explanation.

In an embodiment illustrated in Figs. 5 and 6, the absorbent core 14a of the absorbent member 14 includes a bending-guide portion 30 extending in the longitudinal direction; and the bending-guide portion 30 is in a position overlapping the weakly-joined central region 21 in a planar view (see Fig. 5). The bending-guide portion 30 serves as a bending point where the absorbent core 14a can be bent toward the skin-facing surface side or the non-skin-facing surface side in a cross-sectional view along the width direction Y of the absorbent core 14a. In the present embodiment, the absorbent core 14a includes, as bending-guide portions 30: a central bending-guide portion 31 located in a central area, in the width direction Y, of the absorbent core 14a; and a pair of side bending-guide portions 33 respectively located on both sides, in the width direction Y, of the central bending-guide portion 31. The central bending-guide portion 31 is in a position overlapping the weakly-joined central region 21. In cases where the absorbent core includes a plurality of bending-guide portions 30 as in the present embodiment, it will suffice if at least one of the plurality of bending-guide portions 30 is in a position overlapping the weakly-joined central region 21, and it is preferable that the central bending-guide portion 31 is in a position overlapping the weakly-joined central region 21.

In the present embodiment, since the absorbent core 14a includes the bending-guide portions 31, 33, the absorbent core is configured to bend into a W-shape wherein, in a cross-sectional view, the central bending-guide portion 31 constitutes a mountain portion and the pair of side bending-guide portions 33, 33 constitutes valley portions (see Fig. 6). In this way, the topsheet 12's section overlapping the central bending-guide portion 31, which has been formed into a mountain, can peel off easily, and the section overlapping the central bending-guide portion 31 can easily come into tight contact with depressed sections such as the gluteal cleft and gluteal folds. This thereby improves fittability between the topsheet 12 and the excretion section and peripheral sections thereof. Particularly, since the central bending-guide portion 31, which forms the mountain portion, overlaps the weakly-joined central region 21, the aforementioned tight contact and fittability can be further improved. Even in cases where the absorbent core 14a includes only the central bending-guide portion 31, the absorbent core will bend such that the central bending-guide portion 31 forms a mountain portion, and thus, fittability will be improved.

From the viewpoint of achieving the aforementioned effects more reliably, it is preferable that the arrangement and dimensions of the bending-guide portions 31, 33 are within the following ranges.

In cases where the absorbent core 14a includes the side bending-guide portions 33, it is preferable that the separation distance W12 (see Fig. 5) between one of the side bending-guide portions 33 and the central bending-guide portion 31 is the same as the separation distance between the other side bending-guide portion and the central bending-guide portion. Stated differently, it is preferable that the side bending-guide portions 33 are respectively provided in positions having symmetry with respect to a width-direction bisector CL1 of the absorbent core 14a.

The length L11, L13 (see Fig. 5), in the longitudinal direction X, of each bending-guide portion 31, 33 is preferably 50 mm or greater, more preferably 100 mm or greater, and preferably 350 mm or less, more preferably 300 mm or less, and preferably from 50 to 350 mm, more preferably from 100 to 300 mm.

The width-i.e., the length in the width direction Y-of each bending-guide portion 31, 33 is preferably 20 mm or less, more preferably 15 mm or less. As regards the lower limit thereof, the closer it is to zero, the more preferable; but from the viewpoint of making the absorbent core bend, the width is preferably 1 mm or greater, more preferably 3 mm or greater. The width of each of the bending-guide portions 31, 33 may be the same, or different from one another.

The bending-guide portion 30 may be: a non-fiber-stacked portion wherein the material forming the absorbent core 14a is missing and a space is formed in that section; a sparingly fiber-stacked portion wherein the basis weight of the core-forming material is lower compared to surrounding sections thereof; or a densified portion wherein the core-forming material has been densified. The non-fiber-stacked portion may be a through hole that penetrates the absorbent core 14a, or may be a slit. A slit is a through hole having a relatively narrow width. The densified portion typically forms a depression that has a smaller thickness compared to surrounding sections thereof. The sparingly fiber-stacked portion refers to a section wherein the basis weight of the core-forming material is 50 g/m² or less, and the sparingly fiber-stacked portion may or may not form a depression.

In an embodiment illustrated in Fig. 7, an absorbent core 14b in the absorbent member 14 includes a high-basis-weight region 41, and a low-basis-weight region 43 having a relatively lower basis weight than the high-basis-weight region 41. The low-basis-weight region 43 is provided in the rear portion B (not illustrated), and is in a position overlapping the weakly-joined central region 21 in a planar view. With this configuration, the absorbent core 14b's section in the low-basis-weight region 43 can easily deform along the shape of the buttocks, which thereby improves the fittability between the topsheet 12, which has peeled off, and the buttocks, and allows the skincare agent to be transferred to the excretion section and peripheral sections thereof more easily.

From the viewpoint of achieving the aforementioned effects more reliably, it is preferable that the dimensions and basis weight of the high-basis-weight region 41 and the low-basis-weight region 43 are within the following ranges.

The length L15 (see Fig. 7), in the longitudinal direction X, of the high-basis-weight region 41 is preferably 50% or greater, more preferably 60% or greater, even more preferably 70% or greater, and preferably 90% or less, more preferably 85% or less, even more preferably 80% or less, and preferably from 50 to 90%, more preferably from 60 to 85%, even more preferably from 70 to 80%, with respect to the entire length L4, in the longitudinal direction X, of the absorbent core 14b.

The length L16, in the longitudinal direction X, of the low-basis-weight region 43 is preferably from 10 to 50%, more preferably from 15 to 40%, even more preferably from 20 to 30%, with respect to the entire length L4, in the longitudinal direction X, of the absorbent core 14b.

The basis weight of the high-basis-weight region 41 is preferably 120% or greater, more preferably 140% or greater, and preferably 250% or less, more preferably 230% or less, and preferably from 120 to 250%, more preferably from 140 to 230%, with respect to the basis weight of the low-basis-weight region 43.

The basis weight of the low-basis-weight region 43 is preferably 100 g/m² or greater, more preferably 150 g/m² or greater, even more preferably 200 g/m² or greater, and preferably 600 g/m² or less, more preferably 550 g/m² or less, even more preferably 500 g/m² or less, and preferably from 100 to 600 g/m², more preferably from 150 to 550 g/m², even more preferably from 200 to 500 g/m². On the other hand, the basis weight of the high-basis-weight region 41 is preferably from 150 to 900 g/m², more preferably from 200 to 850 g/m², even more preferably from 250 to 800 g/m², on condition that the basis weight is higher than the basis weight of the low-basis-weight region 43.

Next, a method suitable for manufacturing an absorbent article of the invention will be described. As described above, the topsheet 12 is provided with a skincare agent. An example of a method for providing a skincare agent to the topsheet 12 may include a method involving: introducing a skincare agent into a tank of an application device (applicator); and coating the topsheet with the skincare agent, while, if necessary, heating the skincare agent to increase flowability. Here, the skincare agent is applied to the skin-facing surface of the topsheet 12, or in cases where the topsheet 12 has a layered structure, the skincare agent is applied to the skin-facing surface of the skin-side fiber layer. Examples of methods for applying the skincare agent may include die coaters, blade coaters, air knife coaters, roll coaters, bar coaters, gravure coaters, rod blade coaters, lip coaters, curtain coaters, and slide bead coating.

From the viewpoint of easily applying the skincare agent, it is preferable that the viscosity of the skincare agent, as measured according to the following method, is from 1 to 5000 mPa·s.

### Viscosity Measurement:

Using a tuning-fork vibration viscometer (the present embodiment exemplarily employs VIBRO VISCOMETER CJV5000 (from A&D Company, Limited)), the viscosity is measured in a state where the skincare agent is to be applied to the topsheet. More specifically, the viscosity of the skincare agent is measured at a temperature of 36°C. In cases where the skincare agent is to be applied while being heated to a predetermined temperature, the measurement is performed at the predetermined temperature. Measurement is repeated three times, and the average value is employed as the viscosity.

As described above, a known joining means can be used for joining the topsheet 12 and a member in contact with the topsheet 12. In cases of using an adhesive for joining, the adhesive is applied to the non-skin-facing surface of the topsheet 12. The adhesive may be applied according to a so-called solid fill pattern wherein the adhesive is applied so that there is no non-application section, or may be applied according to an application pattern wherein non-application sections are present. Examples of such application patterns include spiral-shaped, summit-shaped, omega-shaped, curtain-shaped and stripe-shaped patterns. For the adhesive application method, it is possible to employ one of the methods exemplified as application methods for the skincare agent.

The present invention has been described above according to preferred embodiments thereof, but the present invention may employ the foregoing embodiments in combination. For example, the diaper 100 may include an absorbent core including: the bending-guide portions 30 of the embodiment illustrated in Figs. 5 and 6; and the high-basis-weight region 41 and the low-basis-weight region 43 of the embodiment illustrated in Fig. 7.

The present invention is not limited to the foregoing embodiments, and may be modified as appropriate. For example, in the embodiment illustrated in Figs. 1 to 4, in the weakly-joined central region 21 and the firmly-joined side regions 23, the topsheet 12 is joined to the absorbent member 14, which is in contact with the non-skin-facing surface of the topsheet 12. However, the topsheet 12 may be joined to a member other than the absorbent member 14. For example, in cases where a sublayer sheet, which is constituted by a nonwoven fabric for example, is provided between the topsheet 12 and the absorbent member 14, the topsheet 12 may be joined to the sublayer sheet.

The foregoing embodiments are examples wherein the absorbent article of the present invention is applied to a pull-on disposable diaper, but the present invention is not limited thereto, and is also applicable to an open-type disposable diaper. Further, the present invention is applicable to absorbent articles other than disposable diapers, such as sanitary napkins and incontinence pads.

### Industrial Applicability

The absorbent article of the invention offers excellent transferability of a skincare agent to excretion sections, such as the anal area and genital area, and to peripheral sections thereof.

## Claims

1. An absorbent article comprising:
a topsheet (12) that is joined, on a non-skin-facing surface thereof, to a member (14) in contact with the non-skin-facing surface, wherein:
a region, within the topsheet's non-skin-facing surface, that is joined to the member (14) in contact with the non-skin-facing surface includes
a weakly-joined central region (21) located in a central area, in a width direction (Y), of the topsheet (12), and having a relatively low joining strength, and
a pair of firmly-joined side regions (23) respectively located in side areas, in the width direction (Y), of the topsheet (12) and adjacent to the weakly-joined central region (21), and having a relatively high joining strength, wherein the joining strength is measured according to the method of the description;
wherein in the weakly-joined central region (21), the topsheet (12) is peelable from the member (14) in contact with the topsheet (12) according to a crease-flex test evaluation; and in the firmly-joined side regions (23), the topsheet (12) is not peelable from the member (14) in contact with the topsheet (12) according to abrasion under same conditions, wherein the crease-flex test evaluation is performed according to the method of the description; and
a skincare agent is provided to the topsheet (12).

2. The absorbent article according to claim 1, wherein the topsheet (12) is provided with the skincare agent in a region overlapping the weakly-joined central region (21).

3. The absorbent article according to claim 1 or 2, wherein a difference in joining strength, when dry, between the weakly-joined central region (21) and the firmly-joined side region (23) is 0.5 N/50 mm or greater.

4. The absorbent article according to claim 3, wherein the difference in joining strength, when dry, between the weakly-joined central region (21) and the firmly-joined side region (23) is 0.7 N/50 mm or greater and 2.3 N/50 mm or less.

5. The absorbent article according to any one of claims 1 to 4, wherein:
a hot-melt adhesive is applied to each of the weakly-joined central region (21) and the firmly-joined side regions (23); and
when a basis weight of the hot-melt adhesive in the weakly-joined central region (21) is defined as B1 and a basis weight of the hot-melt adhesive in each of the firmly-joined side regions (23) is defined as B2, a value of B2/B1, which is a ratio of B2 to B1, is 1.2 or greater.

6. The absorbent article according to claim 5, wherein, when the basis weight of the hot-melt adhesive in the weakly-joined central region (21) is defined as B1 and the basis weight of the hot-melt adhesive in each of the firmly-joined side regions (23) is defined as B2, the value of B2/B1, which is the ratio of B2 to B1, is 1.3 or greater and 2.5 or less.

7. The absorbent article according to any one of claims 1 to 5, wherein a hot-melt adhesive is applied to the weakly-joined central region (21), and heat sealing is applied to the firmly-joined side regions (23).

8. The absorbent article according to any one of claims 1 to 7, wherein:
the absorbent article further comprises an absorbent core (14a);
the absorbent core (14a) includes a bending-guide portion (30) extending in the longitudinal direction; and
the bending-guide portion (30) is in a position overlapping the weakly-joined central (21) region in a planar view.

9. The absorbent article according to any one of claims 1 to 8, wherein:
the absorbent article further comprises an absorbent core (14b);
the absorbent article includes a front portion (A) to be arranged on the wearer's front side, a crotch portion (C) to be arranged in the wearer's crotch section, and a rear portion (B) to be arranged on the wearer's rear side;
the absorbent core (14b) includes a high-basis-weight region (41), and a low-basis-weight region (43) having a relatively lower basis weight than the high-basis-weight region (41); and
the low-basis-weight region (43) is provided in the rear portion (B) and is in a position overlapping the weakly-joined central region (21) in a planar view.

10. The absorbent article according to any one of claims 1 to 9, wherein:
the topsheet (12) is constituted by two or more layers of fiber layers, including a skin-side fiber layer to be arranged in a position closest to the wearer's skin, and a non-skin-side fiber layer to be arranged in a position farther from the wearer's skin than the skin-side fiber layer; and
the skin-side fiber layer has a higher fiber density than the non-skin-side fiber layer, and is provided with the skincare agent.

## Patentansprüche

1. Absorbierender Artikel, der aufweist:
eine oberste Lage (12), die auf einer nicht-hautzugewandten Oberfläche davon mit einem Element (14) in Kontakt mit der nicht-hautzugewandten Oberfläche verbunden ist, wobei: ein Bereich innerhalb der nicht-hautzugewandten Oberfläche der obersten Lage, der mit dem Element (14) in Kontakt mit der nicht-hautzugewandten Oberfläche verbunden ist, aufweist:
einen schwach verbundenen mittleren Bereich (21), der sich in einem mittleren Bereich in einer Breitenrichtung (Y) der obersten Lage (12) befindet und eine relativ geringe Verbindungsfestigkeit aufweist, und
ein Paar fest verbundene Seitenbereiche (23), die jeweils in Seitenbereichen in der Breitenrichtung (Y) der obersten Lage (12) und benachbart zu den schwach verbundenen mittleren Bereich (21) angeordnet sind und eine relativ hohe Verbindungsfestigkeit aufweisen, wobei die Verbindungsfestigkeit gemäß dem Verfahren der Beschreibung gemessen wird;
wobei in dem schwach verbundenen mittleren Bereich (21) die oberste Lage (12) von dem Element (14), das mit der obersten Lage (12) in Kontakt steht, gemäß einer Knitter-Biege-Testbewertung abziehbar ist; und in den fest verbundenen Seitenbereichen (23) die oberste Lage (12) von dem Element (14), das mit der obersten Lage (12) in Kontakt steht, gemäß einem Abrieb unter denselben Bedingungen nicht abziehbar ist, wobei die Knitter-Biege-Testbewertung gemäß dem Verfahren der Beschreibung durchgeführt wird;
und
ein hautpflegendes Mittel auf der obersten Lage (12) aufgebracht ist.

2. Absorbierender Artikel nach Anspruch 1, wobei die oberste Lage (12) mit dem Hautpflegemittel in einem Bereich versehen ist, der den schwach verbundenen mittleren Bereich (21) überlappt.

3. Absorbierender Artikel nach Anspruch 1 oder 2, wobei die Differenz der Verbindungsfestigkeit im trockenen Zustand zwischen dem schwach verbundenen mittleren Bereich (21) und dem fest verbundenen Seitenbereich (23) 0,5 N/50 mm oder mehr beträgt.

4. Absorbierender Artikel nach Anspruch 3, wobei die Differenz der Verbindungsfestigkeit im trockenen Zustand zwischen dem schwach verbundenen mittleren Bereich (21) und dem fest verbundenen Seitenbereich (23) 0,7 N/50 mm oder mehr und 2,3 N/50 mm oder weniger beträgt.

5. Absorbierender Artikel nach einem der Ansprüche 1 bis 4, wobei:
ein Schmelzklebstoff auf jeden der schwach verbundenen mittleren Bereiche (21) und der fest verbundenen Seitenbereiche (23) aufgetragen ist; und
wenn ein Flächengewicht des Schmelzklebstoffs in dem schwach verbundenen mittleren Bereich (21) als B1 definiert ist und ein Flächengewicht des Schmelzklebstoffs in jedem der fest verbundenen Seitenbereiche (23) als B2 definiert ist, ein Wert von B2/B1, der ein Verhältnis von B2 zu B1 ist, 1,2 oder größer ist.

6. Absorbierender Artikel nach Anspruch 5, wobei, wenn das Flächengewicht des Schmelzklebstoffs in dem schwach verbundenen mittleren Bereich (21) als B1 definiert ist und das Flächengewicht des Schmelzklebstoffs in jedem der fest verbundenen Seitenbereiche (23) als B2 definiert ist, der Wert von B2B 1, der das Verhältnis von B2 zu B1 ist, 1,3 oder größer und 2,5 oder kleiner ist.

7. Absorbierender Artikel nach einem der Ansprüche 1 bis 5, wobei ein Schmelzklebstoff auf den schwach verbundenen mittleren Bereich (21) und eine Heißklebung auf die fest verbundenen Seitenbereiche (23) aufgebracht ist.

8. Absorbierender Artikel nach einem der Ansprüche 1 bis 7, wobei:
der absorbierende Artikel ferner einen absorbierenden Kern (14a) aufweist;
der absorbierende Kern (14a) einen Biegeführungsabschnitt (30) aufweist, der sich in der Längsrichtung erstreckt; und
der Biegeführungsabschnitt (30) sich in einer Position befindet, die den schwach verbundenen mittleren Bereich (21) in einer ebenen Ansicht überlappt.

9. Absorbierender Artikel nach einem der Ansprüche 1 bis 8, wobei:
der absorbierende Artikel ferner einen absorbierenden Kern (14b) aufweist;
der absorbierende Artikel einen vorderen Abschnitt (A), der an der Vorderseite des Trägers anzuordnen ist, einen Schrittabschnitt (C), der im Schrittbereich des Trägers anzuordnen ist, und einen hinteren Abschnitt (B), der an der Rückseite des Trägers anzuordnen ist, aufweist;
der absorbierende Kern (14b) einen Bereich (41) mit hohem Flächengewicht und einen Bereich (43) mit niedrigem Flächengewicht aufweist, der ein relativ geringeres Flächengewicht als der Bereich (41) mit hohem Flächengewicht aufweist; und
der Bereich (43) mit niedrigem Flächengewicht in dem hinteren Abschnitt (B) vorgesehen ist und sich in einer Position befindet, die den schwach verbundenen mittleren Bereich (21) in einer ebenen Ansicht überlappt.

10. Absorbierender Artikel nach einem der Ansprüche 1 bis 9, wobei:
die oberste Lage (12) aus zwei oder mehr Faserschichten besteht, die eine hautseitige Faserschicht, die in einer Position angeordnet ist, die der Haut des Trägers am nächsten ist, und einer nicht-hautseitigen Faserschicht aufweisen, die in einer Position angeordnet ist, die weiter von der Haut des Trägers entfernt ist als die hautseitige Faserschicht; und
die hautseitige Faserschicht eine höhere Faserdichte aufweist als die nicht-hautseitige Faserschicht und mit dem Hautpflegemittel versehen ist.

## Revendications

1. Article absorbant, comprenant :
une feuille supérieure (12) raccordée, sur une surface non exposée à la peau, à un élément (14) en contact avec la surface non exposée à la peau, où :
une zone, à l'intérieur de la surface non exposée à la peau de la feuille supérieure, raccordée à l'élément (14) en contact avec la surface non exposée à la peau, comprend une zone centrale faiblement jointive (21) située dans une surface centrale, dans le sens de la largeur (Y) de la feuille supérieure (12), et ayant une force de liaison relativement faible, et
une paire de zones latérales fortement jointives (23) situées dans des zones latérales respectives, dans le sens de la largeur (Y) de la feuille supérieure (12) et adjacentes à la zone centrale faiblement jointive (21), et ayant une force de liaison relativement élevée, ladite force de liaison étant mesurée selon le procédé de la description ;
où, dans la zone centrale faiblement jointive (21), la feuille supérieure (12) est pelable de l'élément (14) en contact avec la feuille supérieure (12) suivant une évaluation de test de pliage-flexion ; et dans les zones latérales fortement jointives (23), la feuille supérieure (12) n'est pas pelable de l'élément (14) en contact avec la feuille supérieure (12) en fonction de l'abrasion dans les mêmes conditions, l'évaluation de test de pliage-flexion étant effectuée selon le procédé de la description ;
et où
un agent de soin de la peau est prévu dans la feuille supérieure (12).

2. Article absorbant selon la revendication 1, où la feuille supérieure (12) est pourvue d'un agent de soin de la peau dans une zone chevauchant la zone centrale faiblement jointive (21).

3. Article absorbant selon la revendication 1 ou la revendication 2, où la différence de force de liaison, à sec, entre la zone centrale faiblement jointive (21) et la zone latérale fortement jointive (23) est supérieure ou égale à 0,5 N/50 mm.

4. Article absorbant selon la revendication 3, où la différence de force de liaison, à sec, entre la zone centrale faiblement jointive (21) et la zone latérale fortement jointive (23) est supérieure ou égale à 0,7 N/50 mm et inférieure ou égale à 2,3 N/50 mm.

5. Article absorbant selon l'une des revendications 1 à 4, où :
un adhésif thermofusible est appliqué sur la zone centrale faiblement jointive (21) et sur les zones latérales fortement jointives (23) ; et où,
lorsqu'un poids de base de l'adhésif thermofusible dans la zone centrale faiblement jointive (21) est défini comme B1 et qu'un poids de base de l'adhésif thermofusible dans chacune des zones latérales fortement jointives (23) est défini comme B2, une valeur de B2/B 1, qui est un rapport de B2 à B 1, est supérieure ou égale à 1,2.

6. Article absorbant selon la revendication 5, où, lorsque le poids de base de l'adhésif thermofusible dans la zone centrale faiblement jointive (21) est défini comme B 1 et que le poids de base de l'adhésif thermofusible dans chacune des zones latérales fortement jointives (23) est défini comme B2, la valeur de B2/B1, qui est le rapport entre B2 et B1, est supérieure ou égale à 1,3 et inférieure ou égale à 2,5.

7. Article absorbant selon l'une des revendications 1 à 5, où un adhésif thermofusible est appliqué à la zone centrale faiblement jointive (21), et un thermoscellage est appliqué aux zones latérales fortement jointives (23).

8. Article absorbant selon l'une des revendications 1 à 7, où :
l'article absorbant comprend en outre une âme absorbante (14a) ;
l'âme absorbante (14a) comprend une partie de guidage de pliage (30) s'étendant dans le sens de la longueur ; et
la partie de guidage de pliage (30) est à un emplacement de chevauchement de la zone centrale faiblement jointive (21) dans une vue en plan.

9. Article absorbant selon l'une des revendications 1 à 8, où :
l'article absorbant comprend en outre une âme absorbante (14b) ;
l'article absorbant comprend une partie avant (A) à disposer sur le côté avant de l'utilisateur, une partie d'entrejambe (C) à disposer dans la section d'entrejambe de l'utilisateur, et une partie arrière (B) à disposer sur le côté arrière de l'utilisateur ;
l'âme absorbante (14b) comprend une zone à poids de base élevé (41) et une zone à faible poids de base (43) ayant un poids de base relativement inférieur à celui de la zone à poids de base élevé (41) ; et
la zone à faible poids de base (43) est prévue dans la partie arrière (B) et se trouve à un emplacement de chevauchement de la zone centrale faiblement jointive (21) dans une vue en plan.

10. Article absorbant selon l'une des revendications 1 à 9, où :
la feuille supérieure (12) est constituée de deux ou de plusieurs couches de fibres, comprenant une couche de fibres côté peau à disposer dans une position la plus proche de la peau de l'utilisateur, et une couche de fibres côté distant de la peau à disposer dans une position plus éloignée de la peau de l'utilisateur que la couche de fibres côté peau ; et où
la couche de fibres côté peau a une densité de fibres plus élevée que la couche de fibres côté distant de la peau, et est pourvue d'un agent de soin de la peau.
